# EUROPEAN PATENT APPLICATION

(11) **EP 1 516 627 A1**
(43) Date of publication of application: **23.03.2005**
(21) Application number: 03021005.8
(22) Date of filing: 17.09.2003
(51) Int. Cl.: A61K 38/21, A61P 29/00, A61P 37/08, A61P 11/06

(54) **Interferon-Gamma for the treatment of diseases associated with the NOD2 gene**

(71) Applicant: CONARIS research institute AG, 24118 Kiel (DE)
(72) Inventor: Schreiber, Stefan Uni-klinikum Schleswig-Holstein, 24105 Kiel (DE); Seegert, Dirk, 24161 Altenholz (DE); Rosenstiel, Philip, 24116 Kiel (DE)
(74) Representative: Schüssler, Andrea, Dr.

(57) **Abstract**

Described is the use of IFN-γ for the preparation of a medicament for the treatment/prevention of diseases associated with one or more mutations affecting the NOD2 gene or its expression, in particular diseases like asthma, allergy or inflammatory diseases.

## Description

The present invention relates to the use of IFN-γ fo the preparation of a medicament for the treatment/prevention of diseases associated with one or more mutations affecting the NOD2 gene or its expression, in particular diseases like asthma, allergy or inflammatory diseases.

A fundamental for the survival of multicellular organisms is the removal of infectious agents by the host. Specialized host-cell receptors that recognize molecules expressed exclusively by microorganisms are used by animals and plants for the initial detection of microbial antigenes. In animals, detection of microbial agents is mediated by the recognition of pathogen-associated molecular patterns (PAMPs) by specific host pattern-recognition receptors (PRRs). The identification and characterization of plasma membrane TOLL-LIKE RECEPTORS (TLRs) as PRRs have provided fundamental insights into the mechanisms of host defence in animals. This indicates that TLRs have a central role in mediating immune responses to bacterial pathogens. TLRs mediate the secretion of different pro-inflammatory cytokines and cell-surface molecules by activation of the transcription factor nuclear factor kappa B (NF-κB). This leads to a non-specific pathogen resistance and a specific adaptive immunity following clearence of the pathogenic invasion.

Recently, different cytosolic systems have been identified in mammalians which represent a second frontier against invasive bacteria. However, the host factors and signalling pathways that are involved in such recognition are still poorly understood. A constantly growing group of cytosolic proteins comprising a so-called nucleotide-binding oligomerization domain (NOD) has recently been implicated to be involved in bacterial recognition, innate immunity and inflammatory responses. There is evidence that some of these proteins function as PRRs and induce signalling pathways which mediate specific answers against bacteria. More than 20 different members of the NOD family have been described by now. Most of these proteins contain three distinct functional domains, i.e. an N-terminal effector-binding domain (EBD), a central NOD and a C-terminal ligand-recognition domain (LRD). The NOD is required for self-oligomerization which is necessary for the activation of downstream effector molecules (Inohara et al., Oncogene 20 2001),6473. The EBD of NOD proteins is structurally variable and can mediate homophilic as well as non-homophilic protein interaction. Two EBDs, the caspase recruitment domain (CARD) and the pyrin domain (PYD) are involved in homophilic interactions. CARDs and PYDs are found in a large number of NOD proteins that contain C-terminal leucine-rich reapeats (LRR). These proteins build a subfamily of the NOD family called CATERPILLAR (CARD, transcription enhancer, R(purine)-binding, pyrin, lots of LRRs) and includes members such as NOD1, NOD2, IPAF (ICE-protease activating factor) and others.

Almost all human NOD proteins, including NOD1 and NOD2, contain LRRs in their carboxyl termini (Inohara et al., J.Biol.Chem. 274 (1999), 14560; Ogura et al., J.Biol.Chem. 276 (2001), 4812; Bertin et al., J.Biol.Chem. 274 (1999), 12955) and there is evidence that the NOD proteins are negatively regulated through their LRRs. For example, mutant forms of NOD1, NOD2, IPAF and cryopyrin that lack their LRRs have enhanced ability to activate NF-κB or caspase-1 (Inohara et al., J.Biol.Chem. 274 (1999), 14560; Ogura et al., J.Biol.Chem. 276 (2001), 4812; Bertin et al., J.Biol.Chem. 274 (1999), 12955). In addition to the NOD, which mediates self-oligomerization, the amino-terminal EBDs of NOD proteins, including APAF1, CED-4, NOD1, NOD2, IPAF and cryopyrin, have an essential role in linking these proteins to downstream signalling molecules. For example, the CARD of APAF1 interacts with the CARD of pro-caspase-9, whereas the CARD of NOD1 binds to the CARD present in RICK through homophilic CARD-CARD interactions.

Initial studies showed that transient expression of NOD1 and NOD2 by mammalian cells induces NF-κB activation. This activity was not observed with APAF1, indicating that NOD1 or NOD2 and APAF1 activate distinct signalling pathways. Mutation analyses indicated that the CARDs and the NODs of NOD1 and NOD2 were required for the induction of NF-κB whereas its LRRs were dispensable. These observations indicate that the CARDs function as effector domains for NOD1 and NOD2 signalling. Both NOD1 and NOD2 physically associate with RICK, a CARD-containing protein kinase, through homophilic CARD-CARD interactions (Inohara et al., J.Biol.Chem. 274 (1999), 14560; Ogura et al., J.Biol.Chem. 276 (2001), 4812; Bertin et al., J.Biol.Chem. 274 (1999), 12955). A role for RICK in NOD1 and NOD2 signalling is supported by several studies. NOD1 and NOD2 do not activate NF-κB expressed by mouse embryo fibroblasts derived from mice that are deficient in RICK, but NF-κB activity is restored after exogenous addition of RICK. An intermediate region that is located between the CARD and the kinase domain of RICK interacts with IκB kinase (IKK)γ, the regulatory subunit of the IKK complex, thereby linking NOD1 and presumably NOD2 to the IKK. In addition to NF-κB, NOD1 has been reported to activate c-JUN N-terminal kinase (JNK) in response to bacteria.

In addition many NOD proteins, including NOD1, NOD2, IPAF and DEFCAP, promote the activation of caspases. For example, NOD1 promotes activation of caspase-1 in transient overexpression studies (Yoo et al., Biochem.Biophys.Res.Commun. 299 (2002), 652).

Whereas plant R proteins recognize distinct effector molecules from pathogenic bacteria, human TLRs and NOD proteins seem to recognize conserved structures that are shared by many pathogens. NOD1 and NOD2 have been shown to recognize bacterial lipopolysaccharide (LPS) and/or peptidoglycan through their carboxy-terminal LRRs. Peptidoglycan is responsible for providing shape and mechanical rigidity to bacteria. A role for NOD2 in adaptive immune responses is indicated by the observation that MDP functions as an adjuvant for antigen-specific T-cell responses and antibody production. In addition to TNF-α and IL-1β, muramyl dipeptide (MDP) can also induce the secretion of various cytokines, including IL-6 and IL-12, by monocytes and dendritic cells, as well as the expression of membrane-bound co-stimulatory molecules, which promote the differentiation of naive T cells into effector T cells. This indicates that NOD2, which is expressed by monocytes and dendritic cells, has a role in linking innate and adaptive immune responses in a similar manner to that reported for TLRs. MDP synergizes with LPS in the production of cytokines, indicating that TLRs and NOD proteins cooperate in immune responses against bacteria.

Mutations in NOD2 and CIAS1 (the gene that encodes cryopyrin) have been implicated in several autoinflammatory diseases. A frameshift mutation caused by a cytosine insertion that results in an amino acid substitution at position 1007 and a premature stop codon (Leu1007fsinsC) and two missense mutations (Gly908Arg and Arg702Trp) in NOD2 are associated with Crohn's disease, a common inflammatory disease of the intestinal tract (Ogura et al., Nature 411 (2001), 603; Hugot et al., Nature 411 (2001), 599; Hampe et al., Lancet 357 (2001), 1925). Importantly, having one copy of the mutated alleles confers a 2-4-fold increased risk of developing Crohn's disease, whereas homozygocity or compound heterozygocity for NOD2 mutations increases the risk 20-40-fold (Ogura et al., Nature 411 (2001), 603; Hugot et al., Nature 411 (2001), 599; Hampe et al., Lancet 357 (2001), 1925) indicating that lack of NOD2 function is important for disease development. Notably, all three Crohn's disease-associated mutations result in proteins that are defective in the induction of peptidoglycan- and MDP-mediated NF-κB activation. Moreover, activation of NF-κB that is induced by MDP is absent in mononuclear cells from patients with Crohn's disease that are homozygous for the Leu1007fsinsC mutation. The mechanism by which NOD2 mutations increase the susceptibility to Crohn's disease is at present not understood. The observation that the response to bacterial components is impaired in Crohn's disease-associated NOD2 variants indicates that a deficit in sensing bacteria might trigger, secondarily, diffuse activation of NF-κB and inflammation in intestinal tissue through NOD2-independent mechanisms. Alternatively, deficient NOD2 signalling might lead to the inappropriate induction of co-stimulatory signals for T cells and result in the development of inflammatory disease.

The allele frequencies of the three most important mutations of the NOD2 gene in European CD cohorts have been determined as follows: R702W (SNP8): 11%; G908R (SNP12): 6%; Leu1007finsC (SNP13): 12% (Hugot et al., Nature 411 (2001),.599). This means that in total only nearly 30% of all registered CD cases in Europe could be explained by the occurence of one (or more) mutation(s) of the NOD2 gene. Genomewide linkage studies in the past have shown that beside the *IBD1* locus on chromosome 16q12 which contains the NOD2 gene also other loci are in high linkage to CD, i.e. *IBD2* (Chr 12q13), *IBD3* (Chr 6q13), *IBD4* (14q11), *IBD5* (5q31-33) and *IBD6* (19q13) (29-32). However, a second disease gene which is located within this susceptibility regions was not identified until now.

Meanwhile also other diseases have been shown to be associated with one or more polymorphisms of the NOD2 gene. A large population of German schoolchildren (N = 1872) from East and West Germany was genotyped for 3 functional relevant CARD15 polymorphisms for their role in the development of asthma and allergy (Kabesch et al., J. Allergy Clin. Immunol. 111 (2003), 813). Children with the polymorphic allele C2722 had a more than 3-fold risk to develop allergic rhinitis (P <.001) and an almost 2-fold risk for atopic dermatitis (P <.05). Furthermore, the T2104 allele was associated with an almost 2-fold risk for allergic rhinitis (P <.05). When a C insertion at position 3020 was present, the risk of atopy increased by 50% (P <.05) and serum IgE levels were elevated (P <.01). These results indicate that the shared genetic background between Crohn's disease and atopy may impair the recognition of microbial exposures which results in an insufficient downregulation of excessive immune responses, giving rise to either T_{H}2 dominated allergies or T_{H}1 related Crohn's disease.

Crane et al. (Arthritis Rheum 46 (2002), 1629) demonstrated that spondylarthritis in patients with ulcerative colitis (n = 119) (P = 0.016, odds ratio 4.6) was associated with the Gly908Arg polymorphism in the NOD2 gene. This association was not found in a cohort of 78 Crohn's disease patients. The Pro268Ser variant of NOD2 was inversely associated with UC spondylarthritis. The entire study indicates that NOD2 variants may influence the susceptibility to, and manifestation of, colitis spondylarthritis.

Wang et al. (Arthritis Rheum. 46 (2002), 3041) analyzed CARD15 gene in 10 families with heritable multi-organ granulomatoses, including the original Blau syndrome kindred as well as other families with related granulomatous conditions. These studies resulted in the identification, in 5 of the families, of 2 sequence variants at position 334 of the gene product (R334W and R334Q). Affected family members from the original Blau syndrome kindred were heterozygous for the R334W missense mutation; mutations at the same position were also observed in several unrelated Blau syndrome families, some of whose phenotypes included large-vessel arteritis and cranial neuropathy. The missense mutations segregated with the disease phenotype in the families, and were not seen in 208 control alleles. The results demonstrate that NOD2 is an important susceptibility gene for Blau syndrome and for other granulomatoses that display phenotypic traits beyond those of classic Blau syndrome.

Table I summarizes the current knowledge concerning the association of NOD2 mutations with different diseases.

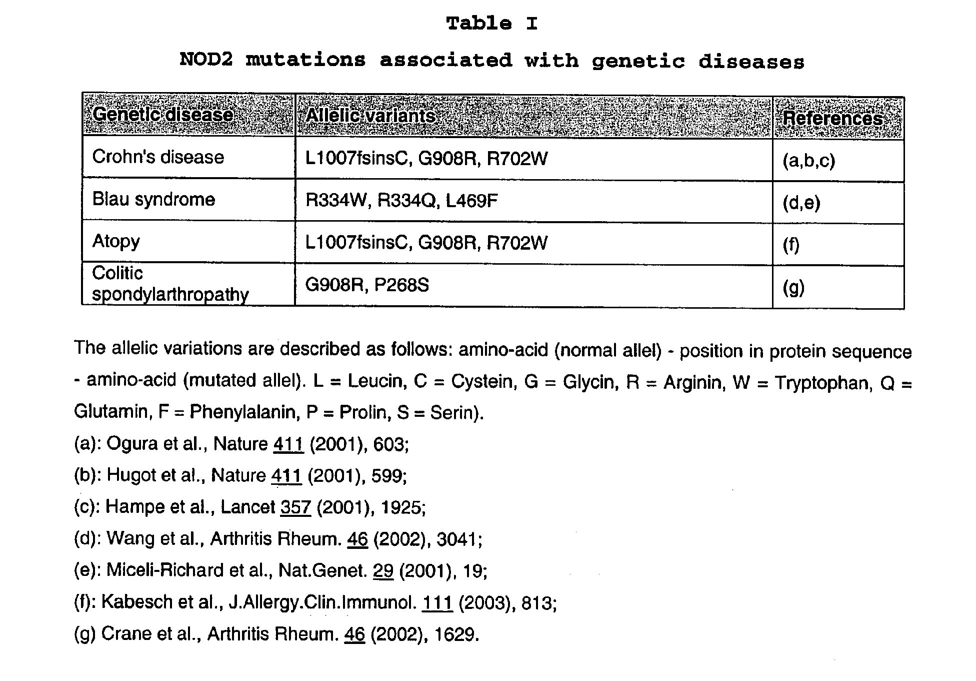

Unfortunately, so far the therapy of NOD2 associated diseases like CD is quite unsatisfactory. Standard immunosuppressive medication with glucocorticoids renders 40% of the patients non-responsive and 30-40% become steroid-dependent. Even second (e.g. azathioprine) and third line (e.g. infliximab) therapies can only improve 30-40% of the treated patients. No therapy is presently known that specifically addresses NOD2 variant patients. Thus, the technical problem underlying the present invention was to provide means suitable for treating or preventing diseases associated with malfunction of the NOD2 gene and thereby targeting a specific therapy to a defined subgroup of patients.

The solution of the said technical problem is achieved by providing the embodiments characterized in the claims. During the experiments leading to the present invention it was found that IFN-γ, preferably in combination with TNF-α, activates the promoter of the NOD2 gene. It could be, furthermore, shown that the treatment of patients bearing a heterocygous mutation of the NOD2 gene (i.e. CD patients) with IFN-γ has a beneficial therapeutic effect, presumably due to the enhanced expression of the unaffected NOD2 allele of the patient: Within one weak of treatment a complete remission was achieved.

### Brief description of the drawings

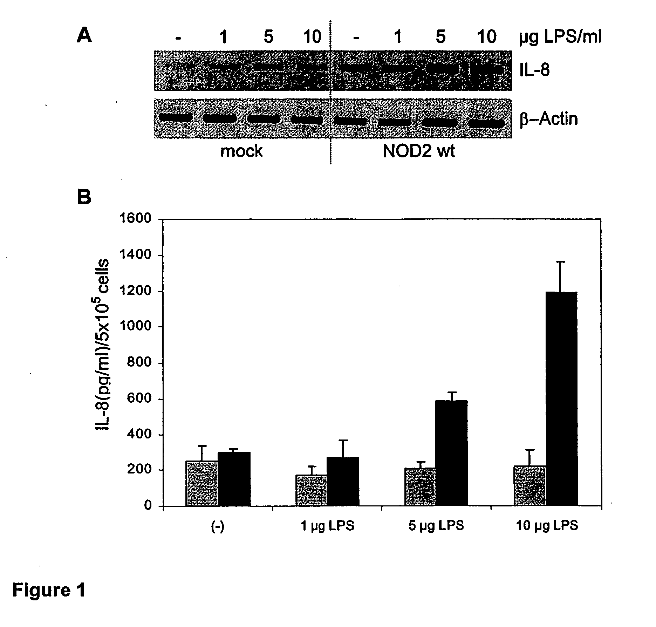

### Figure 1: Production and secretion of interleukin (IL)-8 by SW620 after 24 hours of LPS stimulation

Cells were transfected with 2 *µ*g of either an expression plasmid containing NOD2 (pcDNA3-NOD2) or the appropriate vector control. 8h after transfection, cells were stimulated with LPS. **(A)** 24h post-stimulation mRNA levels for the chemotactic cytokine IL-8 were determined by RT-PCR. Parallel β-actin amplification served as loading control. **(B)** Secretion of IL-8 was detected by ELISA. Values are expressed as mean ± SEM.

### Figure 2: Expression of NOD2 mRNA is synergistically upregulated by co-treatment with TNF-α and IFN-γ

Stimulation of HT-29 or HeLa S3 cells for 20 h with TNF-α (10 ng/ml) and/or IFN-γ (1000 U/ml). β-actin was amplified in parallel in all experiments from the same RNA samples and analyzed on a separate gel (lower panel). The histograms represent means of the densitometrical analysis.

### Figure 3: The NOD2 promoter is synergistically activated by co-treatment with TNF-α and IFN-γ

HeLa S3 cells were transfected with the respective reporter gene plasmid comprising the NOD2 promoter (-1316 to +51) and 24 hours were induced with the cytokines as outlined in the graph. Each experiment was performed in triplicets.

Thus, the present invention relates to the use of interferon-γ for the preparation of a pharmaceutical composition for the prevention or treatment of a disease associated with a heterocygous mutation of the NOD2 gene or decreased expression of the NOD2 gene.

Preferably, in said pharmaceutical composition, interferon-γ is combined with a pharmaceutically acceptable carrier. "Pharmaceutically acceptable" is meant to encompass any carrier, which does not interfere with the effectiveness of the biological activity of the active ingredient and that is not toxic to the host to which it is administered. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and can be administered to the subject at an effective dose.

An "effective dose" refers to an amount of the active ingredient that is sufficient to affect the course and the severity of the disease, leading to the reduction or remission of such pathology.

An "effective dose" useful for treating and/or preventing these diseases or disorders may be determined using methods known to one skilled in the art (see for example, Fingl et al., The Pharmocological Basis of Therapeutics, Goodman and Gilman, eds. Macmillan Publishing Co., New York, pp. 1-46 ((1975)).

Administration of the suitable compositions may be effected by different ways, e.g. by intravenous, intraperetoneal, subcutaneous, intramuscular, topical or intradermal administration. The route of administration, of course, depends on the kind of therapy and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind of therapy, general health and other drugs being administered concurrently.

The use of interferon-γ of the present invention also comprises the administration of DNA sequences encoding said compound in such a form that they are expressed in the patient or a desired tissue of the patient.

The recombinant vectors for expression of interferon-γ can be constructed according to methods well known to the person skilled in the art; see, e.g., Sambrook, Molecular Cloning A Laboratory Manual, Cold Spring Harbor Laboratory (1989). Preferred recombinant vectors useful for gene therapy are viral vectors, e.g. adenovirus, herpes virus, vaccinia, or, more preferably, an RNA virus such as a retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotide encoding a sugar, a glycolipid, or a protein, preferably an antibody. Those skilled in the art know additional methods for generating target specific vectors. Further suitable vectors and methods for in vitro- or in vivo- gene therapy are described in the literature and are known to the persons skilled in the art; see, e.g., WO 94/29469 or WO 97/00957.

In order to achieve expression only in the target organ, the DNA sequence encoding interferon-γ can also be operably linked to a tissue specific promoter and used for gene therapy. Such promoters are well known to those skilled in the art (see e.g. Zimmermann et al, (1994) Neuron 12, 11-24; Vidal et al., (1990) EMBO J. 9, 833-840; Mayford et al., (1995), Cell 81, 891-904; Pinkert et al., (1987) Genes & Dev. 1, 268-76).

Preferably, interferon-γ is used in combination with additional cytokines (e.g. interleukin-10), adhesion molecule blockers (e.g. anti-α or β-integrin, anti-ICAM-1), immunosuppressive drugs (e.g. azathioprine, methotrexate, 6-mercaptopurine) or corticosteroids. Due to the specific interaction between interferon-γ and TNF-α, it may be necessary to co-administrate recombinant TNF-α, a substance that would not be normally used in inflammatory conditions.

Heterocygous mutations of the NOD2 gene which can be prevented/treated by the use of interferon-γ comprise the following mutations: (a) P268S, (b) N289S, (c) R334W, (d) R334Q, (e) H352R, (f) A432V, (g) L468F, (h) A471C, (i) P668L, (j) R684W, (k) R702W, (l) R703C, (m) A725G, (n) R760C, (o) R790Q, (p) G908R, (q) V955I, (r) L1007fsinsC or (s) R1019L.

Preferred medical uses of interferon-γ are the treatment/prevention of an inflammatory disease, preferably Crohn's disease, atopy, spondylarthropathy, or granulomatosis, e.g., Blau syndrome, asthma or allergy, preferably atopic dermatitis or allergic rhinitis.

The below examples explain the invention in more detail.

### Example 1: Material and Methods

### (A) Materials

The expression vector for NOD2 (pcDNA3-NOD2) was obtained from G.Nunez (Ann Arbor, MI, USA) (Ogura et al. 2001, J. Biol. Chem. 276, 7, 4812-18). TNF-α and IFN-γ were purchased from R&D Systems (MN, USA). Purified LPS (protein content <0.2 %) was obtained from Sigma (Deisenhofen, Germany).

### (B) Cell Culture and Cell Transfection

Human epithelial HeLa S3 cells (ACC 161) and intestinal epithelial HT-29 cells (ACC 299) were purchased from the German Collection of Microorganisms and Cell Cultures (DSMZ, Braunschweig, Germany). Another colonic epithelial cell line, SW620 was kindly provided by Holger Kalthoff (UKK, Kiel, Germany). All cells were cultured in RPMI + 10% fetal calf serum (FCS). One day before transfection the cells were seeded at a density of 5 x 10⁵ cells/2 ml on 6-well plates. Transfections were performed with Polyfect^{TM} (Qiagen, Hilden, Germany) according to the manufacturer's manual by using 1.8 *µ*g of the target plasmid and 0.2 *µ*g of the pRL-TK reference plasmid (Promega, CA, USA) for the reporter gene assays. 24 to 48 hours after transfection the cells were harvested and further used for protein or RNA isolation. Transfection efficiency was determined by parallel detection of pRL-TK activity in a dual luciferase reportergene assay (Promega, Madison, WI, USA). Every single transfection experiment was performed in duplicates and was repeated at least three times.

### (C) Reporter gene constructs and mutagenesis

The NOD2 promoter from -1316 to +51 was amplified from 100 ng of human genomic DNA by PCR under standard conditions with the following primers (restriction sites underlined) pNOD2_sense(KpnI) 5'-CGG GTA CCG TGA CAG TTT CAC TGG AGC-3'; pNOD2_anti(*MluI*) 5'-GCA CGC GTA GAA CGC CAA AAG CC-3' and cloned into the pGL3-basic plasmid (Promega). The constructs were sequence-verified with an ABI3700 sequencer (ABI, Foster City, CA, USA) prior to use. All primers were purchased from Eurogentec (Liège, Belgium)

### (D) mRNA isolation and RT-PCR

Total RNA was isolated using the RNeasy kit from Qiagen. 500 ng of total RNA were reverse transcribed as described by Seegert et al., GUT 48 (2001), 326. NOD2-specific primers for PCR: NOD2_sense 5'-CGT TCT GCA CAA GGC CTA CC-3'; NOD2_anti 5'-TCC ATT CGC TTT CAC CGT G-3'. Expected amplicon length: 642 bp. PCR: Denaturation for 5 min at 95°C; 32 cycles (costimulation TNF/IFN) or 39 cycles (kinetics/titration) of 30 sec at 95°C, 20 sec at 60°C, 90 sec at 72°C; final extension for 10 min at 72°C. For IL-8 experiments, the primers were IL-8_s 5'-ACC GGA AGG AAC CAT CTC ACT-3' and IL-8_a 5'-AAA CTT CTC CAC AAC CCT CTG C-3'. To confirm the use of equal amounts of RNA in each experiment all samples were checked in parallel for β-actin mRNA expression. All amplified DNA fragments were analyzed on 1% agarose gels and subsequently documented by a BioDoc Analyzer (Biometra, Göttingen, Germany).

### (E) Dual luciferase reportergene assay

Luciferase activity was determined with a dual luciferase reporter gene kit (DLR) from Promega according to the manufacturer's manual. The cell lysates were analyzed with a MicroLumatPlus LB96V microplate luminometer (EG&G Berthold, Wellesley, MA, USA) after automatic injection of the necessary substrate solutions. All samples were at least measured in duplicates. The results for firefly luciferase activity were normalized to renilla luciferase activity.

### (F) Western blots and patient samples

Western blots were performed as previously described (Waetzig et al., J. Immunol. 168 (2002), 5342). 10 *µ*g of total protein were separated by SDS polyacrylamide gel electrophoresis and transferred to PVDF membrane by standard techniques. NOD2 was detected with an anti-NOD2 antibody (Cayman Chemical Company, Ann Arbor, Michigan, USA) in a dilution of 1:500, which is identical to the antibody used in earlier publications.

### (G)IL-8 enzyme linked immunosorbent assay

Supernatants of IEC cultures transfected with 500 ng of pcDNA3 vector control or pcDNA3NOD2wt vector were collected after 24 hours with or without stimulation with LPS (1-10 *µ*g/ml). IL-8 was measured by enzyme linked immunosorbent assay (ELISA) (R&D Systems, MN, USA) according to the manufacturer's protocol. The results were expressed as pg cytokine/5x10⁵ cells.

### Example 2: NOD2 expression enhances LPS-induced IL-8 secretion in the IEC line SW620

To determine the impact of NOD2 overexpression in epithelial cells, SW620 cells were transfected with 2 µg of either pcDNA3-NOD2 or pcDNA3.1 (mock) as described in Example 1. 8 hours later the cells were treated with different concentrations of purified LPS (1-10 µg/ml). After 24 hours the cells and supernatants were harvested and analyzed for IL-8 mRNA by RT-PCR (Fig. 1A) and secreted IL-8 protein by ELISA (Fig. 1B). Cells transfected with pcDNA3-NOD2 showed significantly elevated levels of IL-8 mRNA when treated with LPS. A dose-dependent increase of secreted IL-8 protein after LPS-stimulation could be detected only in the supernatants of pcDNA3-NOD2 transfected cells. This experiment demonstrates the beneficial role of NOD2 upregulation during bacterial infections. It has also been shown elsewhere that the load of viable intracellular bacteria after *S. typhimurium* infection of Caco2 was significantly higher in cells lacking the NOD2 wildtype protein or cells expressing a mutated NOD2 variant as compared to cells expressing wildtype NOD2.

### Example 3: NOD2 mRNA and protein expression is synergistically upregulated in intestinal epithelial cell lines by TNF-α and IFN-γ

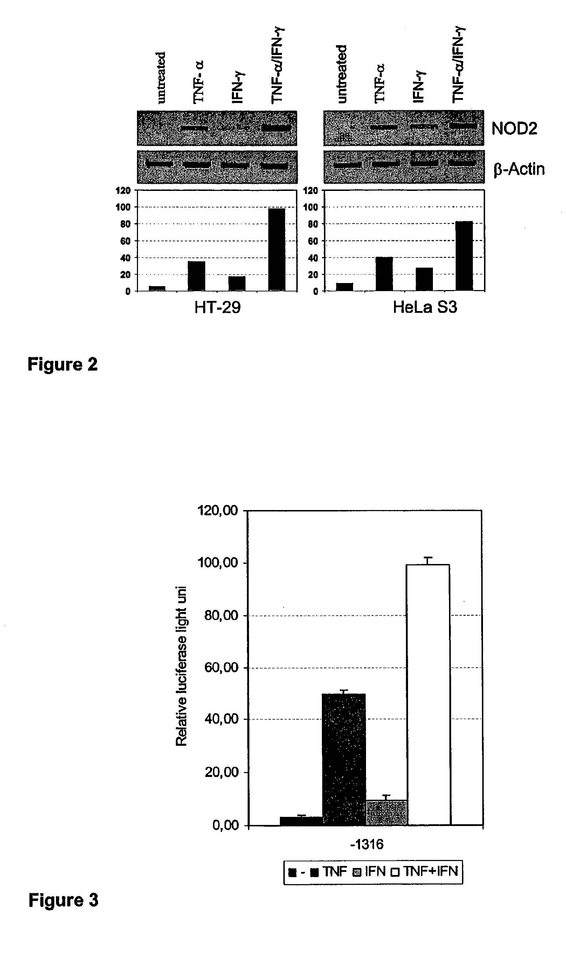

A synergistic upregulation of NOD2 expression effect was seen after co-treatment of HT-29 or HeLa S3 cells with a combination of TNF-α (5 ng/ml) and IFN-γ (1000 U/ml) (Fig. 2A). In untreated cells the amount of NOD2 transcripts was low, IFN-γ alone led to no appreciable increase of NOD2 mRNA expression. The use of equal amounts of total RNA in each experiment was documented by parallel amplification of β-actin mRNA (lower panels). The densitometrical analysis of the bands is represented by the histograms below.

### Example 4: Synergistic activation of the NOD2 promoter by TNF-α and IFN-γ

A luciferase reporter gene construct (pGL3B-NOD2 (-1316)) driven by the NOD2 promoter was transfected into HeLa S3 cells (Figure 3). A 15-fold increase of luciferase activity was observed after treatment with TNF-α alone, whereas cotreatment with TNF-α and IFN-γ resulted into a 30-fold elevated luciferase activity. This experiment clearly demonstrates that IFN-γ enhances the TNF-α-induced expression of the NOD2 gene.

### Example 5: Results of the treatment of Crohn's disease patients bearing a heterocygous mutation of the NOD2 gene

The intention of this experiment was to demonstrate that treatment of Crohn's disease patients bearing a heterocygous mutation of the NOD2 gene has a beneficial therapeutic effect. Based on the experiments shown before the idea of this study was to enhance the expression of the unaffected NOD2 allele of the patient by treatment with IFN-γ. Because Crohn's disease patients are known to express significantly high amounts of TNF-α (and other inflammatory cytokines) application of additional IFN-γ might result in a collaborative induction of the NOD2 gene.

| | |
|---|---|
| Patient | K. P., female, 40 years, 42 kg, 165 cm |
| Diagnosis | Steroid refractory Crohn' disease with heterocygous L1007fsinsC mutation of the NOD2 gene. |
| Substance | Human IFN-γ |
| Application method | Subcutaneous |
| Therapy scheme | 3 times a week, 1.5 *µ*g IFN-γ per kg body weight |

### Result

A complete remission was reached within 1 week of treatment with a significant reduction of the CD activity index (CDAI) from 270 to < 120 (The CDAI is comprising several disease specific parameters, e.g. number of stools, body weight, abdominal pain, endoscopic appearance (Best et al., Gastroenterology 70 (1976), 439. The higher the CDAI the pronounced the inflammation grade. A remission is defined by a CDAI < 150, a severe inflammation > 250.)

## Claims

1. Use of interferon-γ for the preparation of a pharmaceutical composition for the prevention or treatment of a disease associated with a heterocygous mutation of the NOD2 gene or decreased expression of the NOD2 gene.

2. Use according to claim 1, wherein additionally a cytokine, immunosuppressive drug or corticosteroid is used.

3. Use according to claim 2, wherein the additional cytokine is TNF-α.

4. Use according to any one of claims 1 to 3, wherein the NOD2 gene carries one or more of the following mutations: (a) P268S, (b) N289S, (c) R334W, (d) R334Q, (e) H352R, (f) A432V, (g) L468F, (h) A471C, (i) P668L, (j) R684W, (k) R702W, (l) R703C, (m) A725G, (n) R760C, (o) R790Q, (p) G908R, (q) V955I, (r) L1007fsinsC or (s) R1019L.

5. Use according to any one of claims 1 to 4, wherein said disease is an inflammatory disease, asthma or allergy.

6. Use according to claim 5, wherein said inflammatory disease is Crohn's disease, atopy, spondylarthropathy, or granulomatosis.

7. Use according to claim 5, wherein said allergy is atopic dermatitis or allergic rhinitis.

8. Use according to claim 6, wherein said granulomatosis is Blau syndrome.
